# FASCICULE DE BREVET EUROPEEN

(11) **EP 1 846 070 B1**
(45) Date de publication et mention de la délivrance du brevet: **20.12.2017**
(21) Numéro de dépôt: 06709443.3
(22) Date de dépôt: 25.01.2006
(51) Int. Cl.: A61M 15/00

(54) **DISPOSITIF DE DISTRIBUTION DE PRODUIT FLUIDE**
FLUIDPRODUKTABGABEVORRICHTUNG
FLUID PRODUCT DISPENSING DEVICE

(30) Priorité: 25.01.2005 FR 0550215
(43) Date de publication de la demande: 24.10.2007
(60) Demande divisionnaire: 12166070.8
(73) Titulaire: Aptar France SAS, 27110 Le Neubourg (FR)
(72) Inventeur: POCOCK, Andrew Gordon, Cambridge CB10 1SX (GB); KAY, Stuart Brian William, Cambridge CB10 1SX (GB); GREENHALGH, Paul, Cambridge CB10 1SX (GB); O'HARA, Wayne, Cambridge CB10 1SX (GB); DONNETTE, Xavier, 78370 Plaisir (FR)
(74) Mandataire: CAPRI
(86) Numéro de dépôt international: PCT/FR2006/050059
(87) Numéro de publication internationale: WO 2006/079751

(56) Documents cités:
- WO-A-01/26720
- WO-A-02/098495
- WO-A-2004/067069
- WO-A-2005/030305
- US-B1- 6 543 448

## Description

La présente invention concerne un dispositif de distribution de produit fluide, et plus particulièrement un inhalateur de poudre sèche.

Les inhalateurs de poudre sèche sont bien connus dans l'état de la technique. Il en existe différentes sortes. Un premier type d'inhalateur contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Un autre type d'inhalateur consiste à emballer les doses de poudre dans des réservoirs individuels prédosés, puis d'ouvrir un de ces réservoirs à chaque actionnement de l'inhalateur. Cette mise en oeuvre assure une meilleure étanchéité de la poudre, puisque chaque dose n'est ouverte qu'au moment de son expulsion. Pour réaliser ces réservoirs individuels, diverses variantes ont déjà été proposées, telle qu'une bande de blister allongé ou des blisters disposés sur un disque circulaire rotatif. Des inhalateurs comportant des réservoirs individuels, tels que des capsules, qui sont à charger dans l'inhalateur juste avant l'utilisation de celui-ci ont également été décrits dans l'état de la technique. L'avantage de ces dispositifs est qu'il n'est pas nécessaire de stocker l'ensemble des doses à l'intérieur de l'appareil, de sorte que celui-ci peut être de dimension réduite. Par contre évidemment, l'utilisation est plus complexe, puisque l'utilisateur est obligé de charger une capsule dans l'inhalateur avant chaque utilisation. Tous les types d'inhalateurs décrits ci-dessus et existants présentent des avantages et des inconvénients liés à leur structure et à leur fonctionnement. Ainsi, avec certains inhalateurs, se pose le problème de la précision et de la reproductibilité du dosage à chaque actionnement. De même, l'efficacité de la distribution, c'est-à-dire la partie de la dose qui pénètre effectivement dans les poumons de l'utilisateur pour avoir un effet thérapeutique bénéfique, est également un problème qui se présente avec un certain nombre d'inhalateurs. Une solution pour résoudre ce problème spécifique a été de synchroniser l'expulsion de la dose avec l'inhalation du patient. A nouveau, ceci pouvait générer des inconvénients, à savoir que généralement dans ce type de dispositif, la dose est chargée dans un conduit d'expulsion avant l'inhalation, puis l'expulsion est synchronisée avec l'inhalation. Ceci signifie que si l'utilisateur laisse tomber, secoue ou manipule de manière non souhaitée ou inadaptée l'inhalateur entre le moment où il a chargé la dose (soit à partir d'un réservoir multidoses soit à partir d'un réservoir individuel) et au moment où il inhale, il risque de perdre toute ou partie de cette dose, celle-ci pouvant se répartir à l'intérieur de l'appareil. Dans ce cas il peut se présenter un gros risque de surdosage lors de la prochaine utilisation du dispositif. L'utilisateur qui se rendra compte que sa dose n'est pas complète chargera une nouvelle dose dans l'appareil, et lors de l'inhalation de cette nouvelle dose, une partie de la dose précédente perdue dans l'appareil pourrait être alors expulsée en même temps que la nouvelle dose, provoquant un surdosage. Selon les traitements envisagés, ce surdosage peut être très néfaste et c'est une exigence de plus en plus forte des autorités de tous les pays de limiter au maximum ce risque de surdosage. Concernant l'ouverture des réservoirs individuels, il a été proposé de peler ou décoller la couche de fermeture. Ceci présente l'inconvénient d'une maîtrise difficile des forces à appliquer pour garantir une ouverture totale sans risquer d'ouvrir le réservoir suivant, particulièrement si les moyens d'ouverture doivent être actionnés par l'inhalation. En variante, il a été proposé de percer la paroi ou couche de fermeture. Ceci présente l'inconvénient que les parties de paroi découpées risquent de retenir une partie de la dose à l'intérieur du réservoir, la précision et reproductibilité de dosage n'étant donc pas garanties.

La présente invention a pour but de fournir un dispositif de distribution de produit fluide, en particulier un inhalateur de poudre sèche qui ne reproduit pas les inconvénients susmentionnés.

En particulier, la présente invention a pour but de fournir un tel inhalateur qui soit simple et peu coûteux à fabriquer et à assembler, fiable d'utilisation, garantissant une précision de dosage et une reproductibilité du dosage à chaque actionnement, fournissant un rendement optimal quant à l'efficacité du traitement, en permettant de distribuer une part importante de la dose au niveau des zones à traiter, en particulier les poumons, évitant de manière sûre et efficace les risques des surdosages, de dimensions aussi petites que possibles, tout en garantissant une étanchéité et une intégrité absolue de toutes les doses jusqu'à leur expulsion. WO0126720 divulgue un dispositif de distribution de produit fluide, comportant au moins un réservoir individuel contenant une dose unique de poudre, des moyens d'ouverture étant prévus pour ouvrir un réservoir individuel à chaque actionnement du dispositif, permettant à l'utilisateur d'inhaler la dose contenue dans ledit réservoir ouvert, la poudre contenue dans un réservoir ouvert étant expulsée par un écoulement d'air créé par l'inhalation de l'utilisateur, lesdits moyens d'ouverture comportant des moyens de perçage et/ou de coupage adaptés à découper une paroi de fermeture du réservoir lesdits moyens d'ouverture étant déclenchés par l'inhalation de l'utilisateur. La présente invention a donc pour objet un dispositif de distribution de produit fluide selon la revendication 1. Les revendications dépendantes décrivent des modes de réalisation avantageux.

Ces caractéristiques et avantages et d'autres de la présente invention apparaîtront plus clairement au cours de la description détaillée suivante de plusieurs modes et variantes de réalisation de celle-ci, faite en référence aux dessins joints, donnés à titres d'exemples non limitatifs, sur lesquels,
- la figure 1 est une vue schématique en section transversale d'un dispositif de distribution selon un premier mode de réalisation,
- les figures 2a et 2b sont des vues de détails du système de déclenchement par l'inhalation, selon un mode de réalisation avantageux, respectivement sans et pendant l'inhalation,
- la figure 3 est une vue schématique d'un autre mode de réalisation du système de déclenchement par l'inhalation,
- la figure 4 est une vue schématique d'encore un autre mode de réalisation du système de déclenchement par l'inhalation,
- les figures 5a à 8b montrent une séquence d'utilisation du dispositif de la figure 1, en représentant respectivement une vue externe et une vue interne du dispositif pour les étapes respectives de repos (figures 5a et 5b), de chargement ou ouverture du dispositif (figures 6a et 6b), d'inhalation (figures 7a et 7b) et de fin d'utilisation ou fermeture du dispositif (figures 8a et 8b),
- les figures 9 et 10 montrent schématiquement des moyens de blocage selon une première variante de réalisation,
- les figures 11 et 12 montrent une autre variante de réalisation des moyens de blocage,
- les figures 13 à 16 montrent schématiquement des moyens d'ouverture selon un mode de réalisation avantageux, respectivement avant ouverture d'un réservoir, après son ouverture, pendant l'entrée de l'écoulement d'air dans le réservoir et pendant la sortie de l'écoulement air + poudre hors du réservoir,
- la figure 17 représente des moyens de perçage et/ou coupage avantageux,
- la figure 18 représente une variante de réalisation des moyens de perçage et/ou coupage,
- les figures 19 et 20 montrent deux variantes de réalisation des moyens d'ouverture, le réservoir de la figure 19 étant en position d'ouverture,
- la figure 21 représente schématiquement en détail une variante avantageuse des moyens de support mobiles,
- la figure 22 représente schématiquement en détail les moyens de réception de bande usée,
- les figures 23 et 24 montrent schématiquement en détail deux variantes de moyens d'indication de doses,
- la figure 25 montre schématiquement en détail des moyens d'aplatissement des réservoirs vides,
- les figures 26 et 27 sont des vues similaires à la figure 1 et montrent respectivement deux autres modes de réalisation du dispositif de distribution,
- la figure 28 est une vue schématique en perspective d'encore un autre mode de réalisation de l'invention,
- la figure 29 est une vue schématique en perspective d'une autre variante de réalisation des moyens de perçage et/ou coupage,
- la figure 30 est une vue schématique en section transversale des moyens de perçage et/ou coupage de la figure 29,
- la figure 31 est une vue similaire à celle de la figure 29, montrant encore un autre variante de réalisation des moyens de perçage et/ou coupage,
- la figure 32 est une vue schématique en section transversale des moyens de perçage et/ou coupage de la figure 31,
- la figure 33 est une vue schématique partielle en section transversale d'encore un autre mode de réalisation du système de déclenchement par l'inhalation, en position avant inhalation,
- la figure 34 est une vue similaire à celle de la figure 33, après inhalation,
- la figure 35 est une vue schématique en section transversale des moyens de support mobiles, en position de non-distribution,
- la figure 36 est une vue similaire à celle de la figure 35, en position de distribution,
- les figures 37 et 38 sont des vues schématiques respectivement en section et en perspective des moyens de positionnement en rotation coopérant avec les moyens de support mobiles,
- la figure 39 est une vue schématique en section transversale d'une variante de réalisation du diaphragme des figures 33 et 34,
- la figure 40 est une vue de détail du diaphragme de la figure 39,
- la figure 41 est une vue schématique en perspective montrant la fixation de l'embout buccal sur le corps, selon un mode de réalisation particulier, et
- la figure 42 est une vue schématique en section montrant un exemple de moyens de réglage d'écoulement d'inhalation, en position d'inhalation.

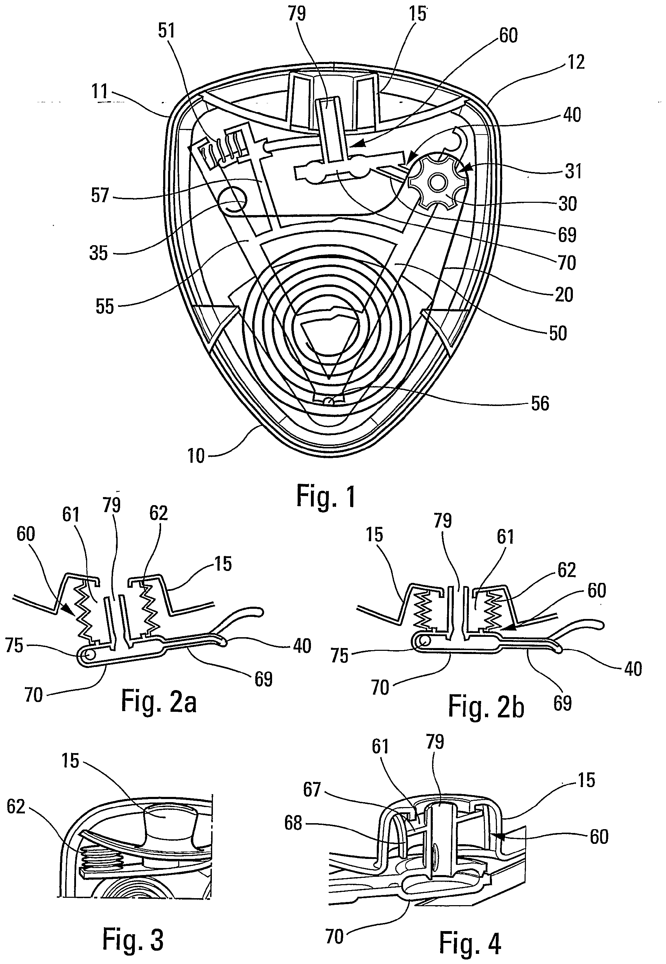

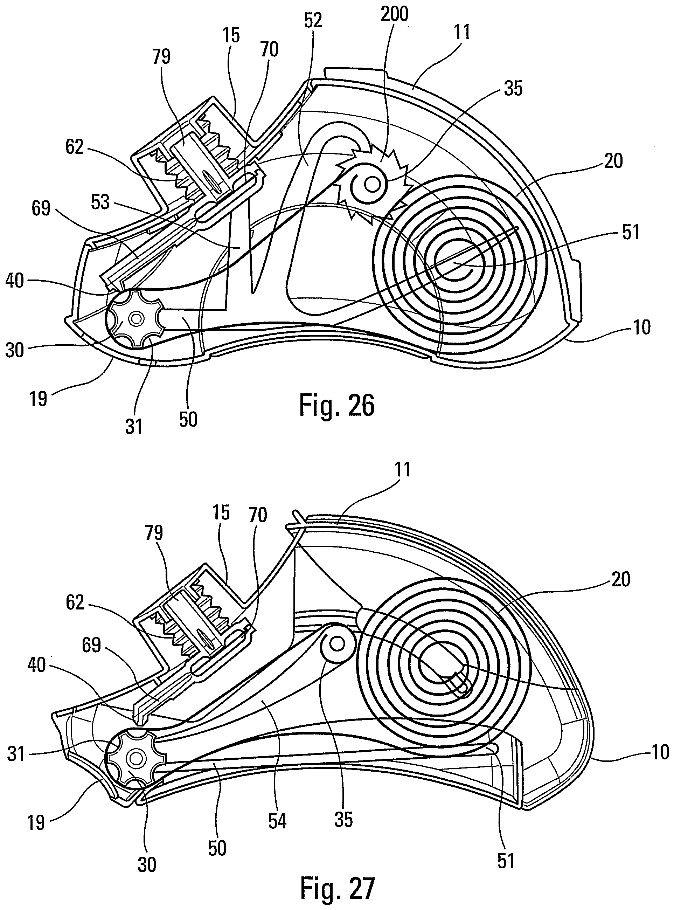

En référence aux figures 1 à 8b, il est représenté un premier mode de réalisation d'un inhalateur de poudre sèche. Cet inhalateur comporte un corps central 10 sur lequel sont montées coulissantes deux parties latérales 11, 12 formant capot lorsque le dispositif est fermé et adaptées à être séparées l'une de l'autre pour ouvrir le dispositif et ainsi charger le dispositif comme cela sera décrit ci-après. Le corps 10 peut être de forme environ arrondie comme représenté sur les figures, mais il pourrait avoir tout autre forme appropriée. Le corps 10 comporte un embout buccal ou d'inhalation 15 à travers lequel l'utilisateur va inhaler lors de l'actionnement du dispositif. Les deux parties latérales 11, 12 formant capot peuvent s'ouvrir par pivotement autour d'un axe de rotation commun comme représenté sur les figures, mais tout autre moyen d'ouverture du dispositif est envisageable. Par ailleurs, on pourrait ne prévoir qu'un seul élément de capot mobile 11 par rapport au corps 10, comme représenté sur les figures 26 et 27.

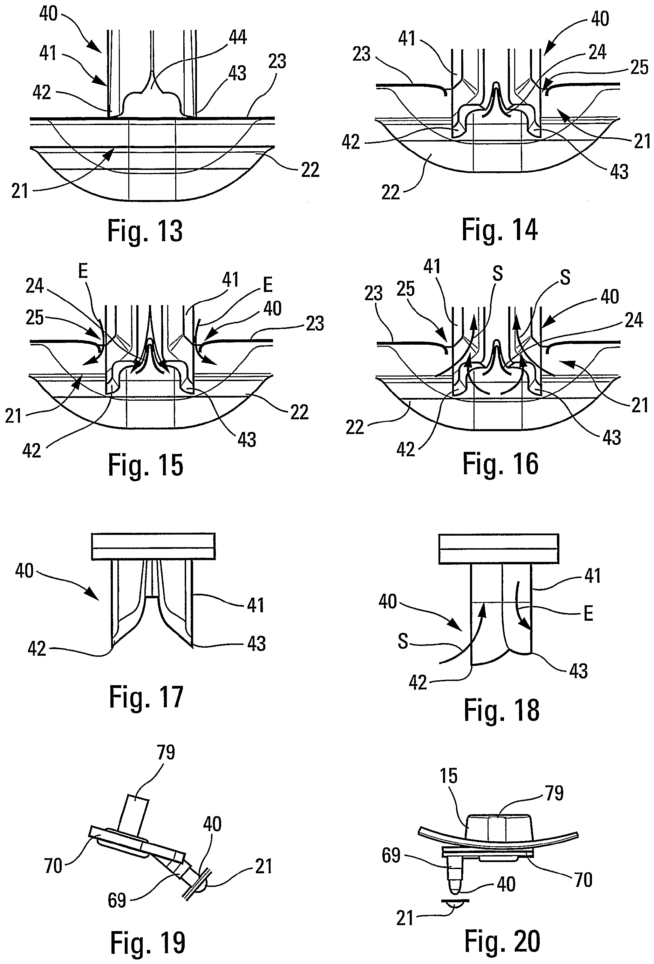

Avantageusement, le corps comporte une fenêtre 19 à travers laquelle le comptage des doses émises ou restant à émettre peut être affiché de manière visible pour l'utilisateur. Cette fenêtre 19 peut avantageusement être prévue sur ou proche de l'axe de rotation des parties latérales 11, 12 formant le capot. A l'intérieur du corps 10, il est prévu une bande 20 de réservoirs individuels 21, également appelés blisters, de préférence une bande allongée 20 sur laquelle les blisters 21 sont disposés les uns derrière les autres, de manière connue. Ces blisters 21 ne sont pas représentés sur les vues d'ensemble des figures 1, 5b, 6b, 7b, 8b, 26 et 27, pour ne pas surcharger ces dessins dans des buts de clarté, mais ils sont visibles sur les figures 13 à 16 et 21 et 23. Cette bande de blister 20 est avantageusement constituée d'une couche ou paroi de base 22 formant les cavités 21 recevant les doses de poudre, et d'une couche ou paroi de fermeture 23 qui obture de manière étanche chacun desdits blisters 21. La bande de blister 20 peut être enroulée à l'intérieur du corps 10 et des moyens d'entraînement de bande 30 sont prévus pour progressivement dérouler cette bande de blister et amener un réservoir individuel ou blister respectif 21 dans une position de distribution à chaque actionnement du dispositif. Lorsqu'un réservoir individuel 21 a été vidé par une inhalation, la partie de bande 35 comportant lesdits réservoirs vides est avantageusement adaptée à s'enrouler dans un autre endroit dudit corps 10 comme cela sera décrit plus en détail ci-après.

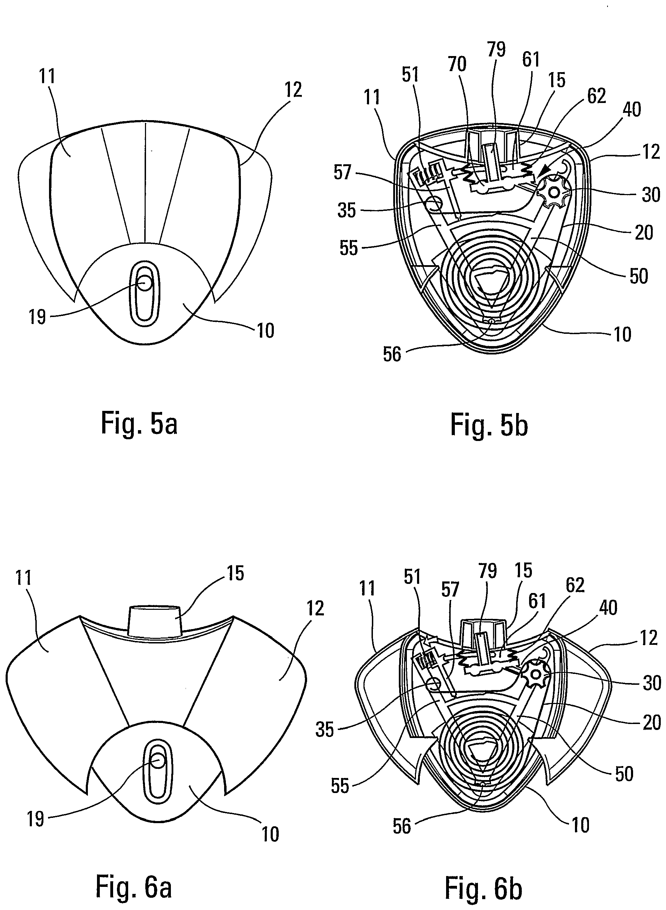

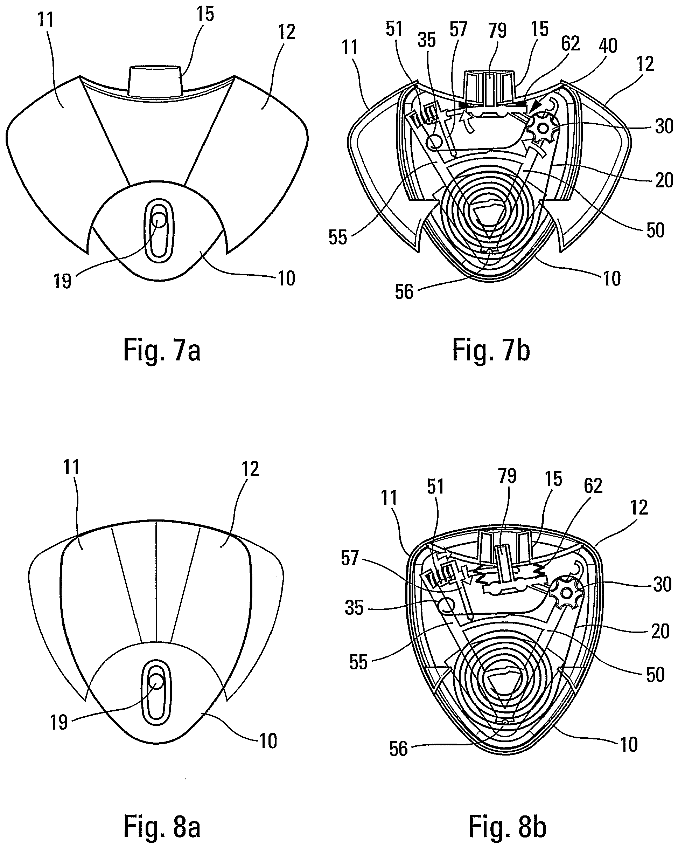

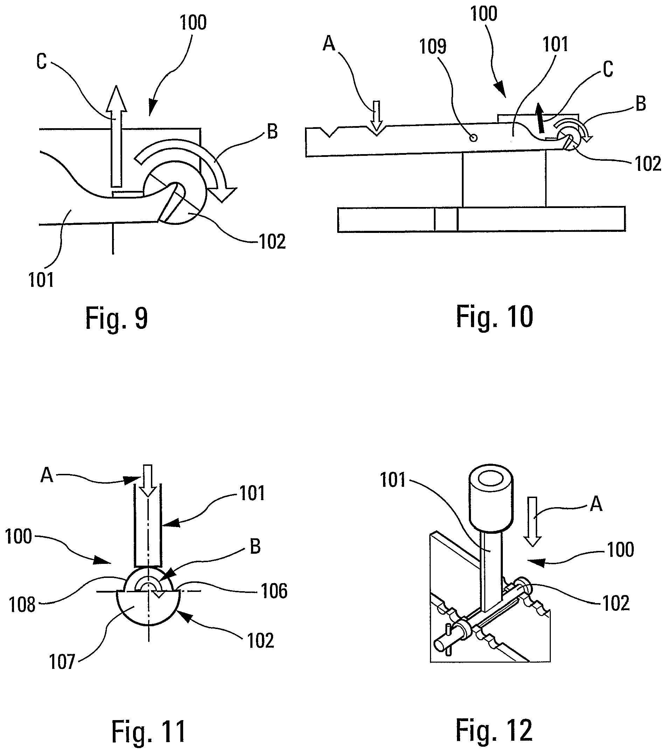

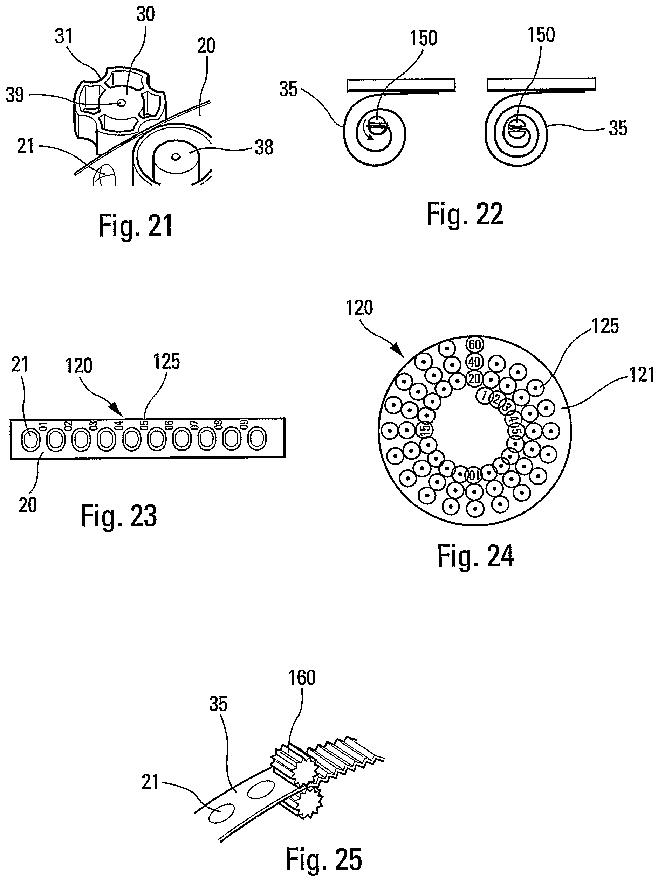

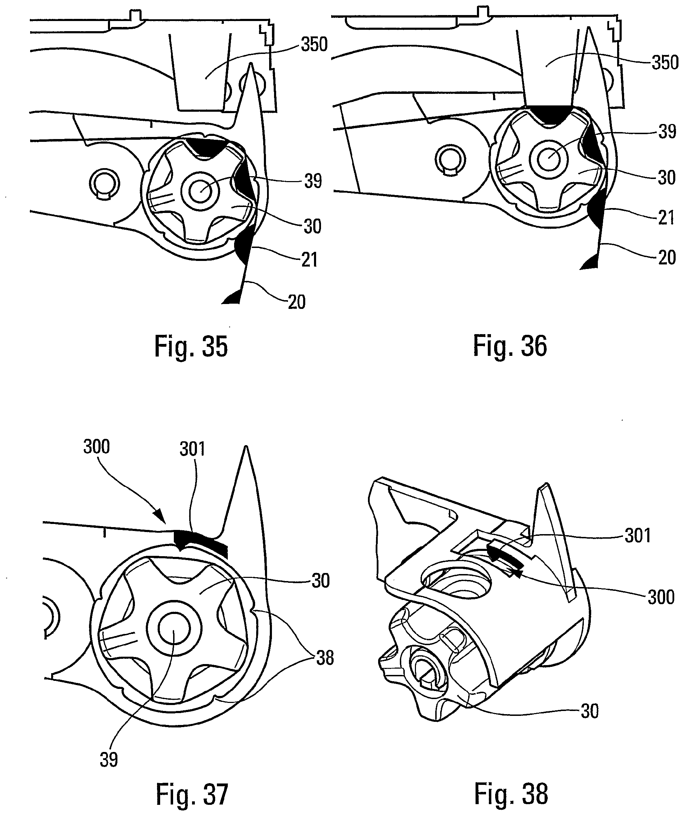

Selon un premier aspect de l'inhalateur, des moyens d'ouverture de réservoir 40 sont prévus dans ou solidaires du corps 10, ces moyens d'ouverture 40 comportant des moyens de perçage et/ou de coupage 41 de la couche de fermeture des blisters. Des moyens de support mobiles 50 sont également prévus dans le dispositif, et sont adaptés à supporter un réservoir donné, qui est destiné à être ouvert lors de la prochaine inhalation. Ces moyens de supports mobiles 50 sont adaptés à déplacer le réservoir à vider contre lesdits moyens de perçage et/ou de coupage 41 du dispositif lors de l'actionnement. Avantageusement, ces moyens de supports mobiles 50 sont sollicités par un élément élastique 51, tel qu'un ressort ou tout autre élément élastique équivalent, ledit élément élastique 51 pouvant être préchargé lors de l'ouverture du dispositif. Avantageusement, les moyens de support mobiles 50 sont déplaçables entre une première position (position de non-distribution) et une seconde position (position de distribution) qui est la position d'ouverture du réservoir. Le mouvement entre ces première et seconde positions est avantageusement réalisé le long d'une direction courbe. En se référant plus particulièrement à l'exemple de réalisation représenté sur les figures 1 à 8, on constate que l'élément de support mobile 50 est solidaire d'une tige 50 articulée par rapport audit corps 10. Une roue de guidage 30 fixée sur ladite tige 50 reçoit et guide les blisters. Une rotation de cette roue de guidage 30 fait avancer la bande de blister 20. Dans une position angulaire particulière, un réservoir donné 21 est toujours en position pour être ouvert par les moyens d'ouverture 40, c'est-à-dire les moyens de perçage et/ou de coupage 41. Avantageusement, des moyens de positionnement en rotation 300 de ladite roue de guidage 30 peuvent être prévus pour déterminer précisément la position angulaire de ladite roue de guidage 30 après chaque rotation. Ces moyens de positionnement 300 peuvent, selon une variante avantageuse représentée sur les figures 37 et 38, comporter une projection ou doigt 301 dont une extrémité coopère élastiquement avec des encoches 38 prévues autour de ladite roue de guidage 30. Avantageusement, les encoches 38 comportent un profil en V qui guide automatiquement ledit doigt 301 vers la position centrale de l'encoche, assurant un positionnement angulaire précis à chaque rotation. La roue de guidage 30 forme de préférence les moyens d'entraînement de bande. Eventuellement, une roue complémentaire 38 pourrait être prévue pour aider un guidage et/ou à l'entraînement de la bande de blisters 20, comme visible sur la figure 21. Ladite tige 50 peut être reliée à une seconde tige 55 de manière à former un V, la pointe du V étant formée par l'axe de pivotement de la ou des tige(s). La seconde tige, qui peut être fixe ou pivotante, peut supporter l'élément élastique 51, tel qu'un ressort, qui coopère également avec un élément actif 57. Les figures 5a à 8b montrent un cycle d'actionnement du dispositif. Les figures 5a et 5b montrent le dispositif en position de repos, fermée. Lors de l'ouverture du dispositif (figures 6a et 6b), les deux parties latérales formant capot 11 et 12 sont écartées l'une de l'autre en pivotant sur le corps 10 pour ouvrir le dispositif. L'élément actif 57 est alors sollicité contre la seconde tige 55 pour comprimer et donc charger le ressort 51. Dans cette position la tige 50 supportant la roue de guidage 30 ne peut pas se déplacer en pivotement car elle est retenue par des moyens de blocage 100 appropriés (non représentés sur les vues d'ensemble, mais visibles sur les figures 9 à 12). C'est lors de l'inhalation par l'utilisateur à travers l'embout buccal 15 (figures 7a et 7b) que ces moyens de blocage 100 seront débloqués, ce qui provoquera alors le pivotement de ladite tige 50 et donc de ladite roue de guidage 30 en direction des moyens d'ouverture 40, et donc l'ouverture d'un réservoir 21 par lesdits moyens de perçage et/ou de coupage 41. La roue de guidage 30 étant fixée sur une tige 50 pivotant autour d'un axe de pivotement, le mouvement du réservoir 21 est donc dans ce mode de réalisation réalisé selon une direction courbe. Cette direction courbe lors de l'ouverture fournit un avantage particulier qui sera décrit plus en détail ci-après.

Avantageusement, des moyens de butée 350 sont prévus pour déterminer précisément la position de distribution de la roue de guidage 30 après chaque inhalation. Ces moyens de butée peuvent comporter un plot 350, comme représenté sur les figures 35 et 36, adapté à coopérer en position de distribution avec une ou des surface(s) plane(s) correspondante(s) de la roue de guidage 30. De préférence, une surface plane est associée à chaque évidement. Dans cet exemple, cette butée 350 participe au positionnement correct en rotation de la roue de guidage 30 au moment où les moyens de perçage et/ou coupage pénètrent dans le réservoir. La butée 350 définit donc non seulement la profondeur de pénétration desdits moyens de perçage et/ou coupage dans le réservoir, mais aussi leur centrage par rapport au réservoir, afin de garantir un expulsion optimale de la poudre et une reproductibilité de la dose élevée à chaque actionnement. Ces moyens de butée 350 peuvent être associés aux moyens de positionnement en rotation 300 susmentionnés de manière à prédéterminer de manière précise chaque position de la roue de guidage, en position de non-distribution, en position de distribution, et aussi lors des déplacements de la roue de guidage 30 entre ces positions. Ceci permet d'éviter des risques de blocage du dispositif en cas de mauvais positionnement de ladite roue de guidage.

Dans l'exemple représenté, lorsque le réservoir 21 se déplace vers sa position d'ouverture pour être ouvert par les moyens de perçage et/ou de coupage 41, ces moyens de perçage et/ou de coupage 41 sont de préférence fixes par rapport au corps. Toutefois, il est envisageable que ces moyens de perçage et/ou de coupage 41 puissent également être mobiles pendant la phase d'ouverture du réservoir 21. Par exemple, les moyens de coupage et/ou de perçage 41 pourraient se déplacer en direction du réservoir 21 pendant que le réservoir 21 se déplace en direction des moyens de perçage et/ou de coupage 41. Dans une autre variante, on pourrait également envisager que le réservoir 21 et les moyens de perçage et/ou de coupage 41 se déplacent dans la même direction lors de l'actionnement, le réservoir 21 se déplaçant plus rapidement dans cette direction de sorte qu'il vient en contact avec lesdits moyens de perçage et/ou de coupage 41 pour être ouvert.

Comme expliqué ci-dessus, il est souhaitable que l'actionnement des moyens d'ouverture soit réalisé par l'inhalation de l'utilisateur. Pour réaliser ce déclenchement par inhalation des moyens d'ouverture de réservoir, on peut prévoir un système de déclenchement par l'inhalation qui comporte avantageusement une unité 60 déplaçable et/ou déformable sous l'effet de l'inhalation, cette unité 60 étant adaptée à libérer les moyens de blocage 100. Cette unité 60 comprend avantageusement une chambre d'air déformable 61 coopérant avec les moyens de blocage 100 desdits moyens de support mobiles 50. L'inhalation de l'utilisateur provoque la déformation de ladite chambre d'air déformable 61, permettant ainsi de libérer lesdits moyens de blocage 100 et donc de débloquer les moyens de support mobiles 50 pour permettre le déplacement de la roue de guidage 30 et d'un réservoir respectif 21 vers sa position d'ouverture. Avantageusement, cette chambre d'air 61 peut comprendre une membrane déformable, tel qu'un soufflet ou poche 62, qui peut être relié d'une part à l'embout buccal 15 et d'autre part auxdits moyens de blocage 100 de manière directe ou indirecte. Ainsi, lors de l'inhalation, le soufflet ou poche 62 se déforme et se contracte provoquant le déplacement desdits moyens de blocage 100 dans une position de déblocage. En variante, le soufflet pourrait être remplacé par une membrane déformable quelconque.

L'inhalateur comporte en outre une chambre de distribution 70 qui est destinée à recevoir la dose de poudre après ouverture d'un réservoir respectif 21. Avantageusement, cette chambre de distribution 70 est pourvue d'au moins une bille 75 qui se déplace à l'intérieur de ladite chambre 70 pendant l'inhalation, pour améliorer la distribution du mélange air et poudre après ouverture d'un réservoir 21, afin d'augmenter l'efficacité du dispositif.

Selon une variante particulière, la chambre d'air déformable 61 coopère avec la chambre de distribution 70. Cette chambre de distribution 70 peut donc être reliée aux moyens d'ouverture 40 du réservoir, et en particulier aux moyens de perçage et/ou de coupage 41, et comporte un orifice de distribution 79. Eventuellement, cette chambre de distribution 70 peut être déplaçable elle-même entre une position de repos et une position d'inhalation, de telle sorte que lorsqu'un utilisateur inhale à travers l'embout buccal 15, en provoquant la déformation de la chambre à air déformable 61, la chambre de distribution 70 se déplace de sa position de repos vers sa position d'inhalation. Dans cette position d'inhalation, l'orifice de distribution 79 vient se placer dans ledit embout buccal 15, pour assurer une bonne distribution de la dose, comme visible en figure 7a. Dans l'exemple représenté sur les figures 2a, 2b, 5b à 8b, 26 et 27, le soufflet 62 est donc connecté d'une part à l'embout buccal 15, et d'autre part à cette chambre de distribution 70, dans le chemin d'écoulement d'inhalation de l'utilisateur. Comme représenté sur les dessins, il peut être avantageux que les moyens d'ouverture 40, en particulier les moyens de perçage et/ou de coupage 41 soient formés directement sur ladite chambre de distribution 70, par exemple à l'extrémité d'un canal 69 menant à ladite chambre 70. De même, cette chambre de distribution 70, dans sa position d'inhalation, peut être adaptée à libérer les moyens de blocage 100 qui retenaient avant les moyens de support mobiles 50 dans la position initiale, afin de permettre à ces moyens de support mobiles 50 de déplacer le réservoir 21 vers la position d'ouverture.

Après l'inhalation, comme visible sur les figures 8a et 8b, lorsque l'utilisateur referme le dispositif, tous les composants reviennent vers leur position de repos initiale, à savoir que les moyens de support mobiles 50 pivotent par rapport à leur axe de pivotement 56 en retour vers leur première position initiale en éloignement des moyens d'ouverture de réservoir 40, et l'élément actif 57 coopérant avec le ressort préchargeable 51 est également ramené dans sa position de repos initiale dans laquelle le ressort 51 n'est pas comprimé. Le dispositif est alors prêt pour un nouveau cycle d'utilisation.

D'autres moyens de déclenchement par l'inhalation pourraient aussi être utilisés en variante, par exemple en utilisant un clapet pivotant qui, lorsque l'utilisateur inhale, pivote sous l'effet de la dépression créée par cette inhalation, le pivotement de ce clapet provoquant la libération des moyens de blocage des moyens de support mobiles et donc le déplacement du réservoir vers les moyens d'ouverture.

La figure 3 montre une variante de réalisation du soufflet 62, dans laquelle le soufflet n'est pas disposé directement entre l'embout buccal 15 et la chambre de distribution 70, mais logé dans le corps principal 10 sous la chambre de distribution 70. Dans cette variante de réalisation, lors de l'inhalation, le soufflet pourrait même se déformer pour se gonfler, et provoquer ainsi le déplacement de la chambre de distribution vers sa position d'inhalation, alors que dans le mode de réalisation précédemment décrit le soufflet se contracte lors de l'inhalation pour tirer la chambre de distribution en direction de sa position d'inhalation.

La figure 4 montre un autre mode de réalisation dans lequel le soufflet (ou la membrane) est remplacé par un piston 67 ou similaire coulissant dans un manchon creux 68 pour déformer la chambre d'air 61. Le piston 67, qui peut être réalisé sous la forme d'une plaque mince peut comporter un trou (non représenté) pour contrôler la résistance à l'écoulement d'air. Dans l'exemple représenté, le piston 67 est solidaire de la chambre de distribution 70 et le manchon 68 est solidaire de l'embout buccal 15, mais l'inverse est aussi envisageable.

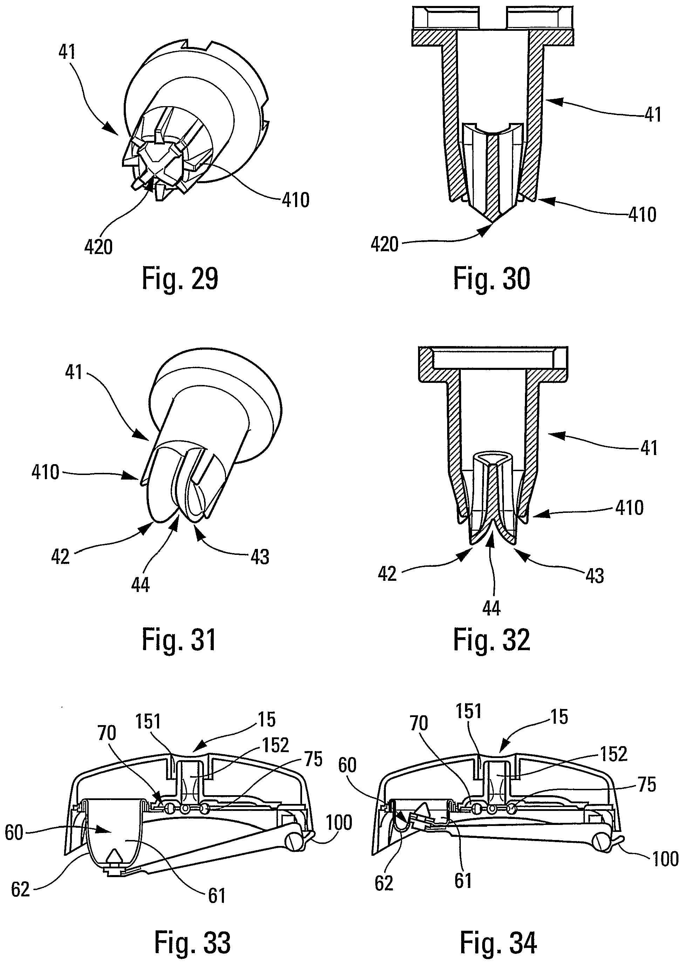

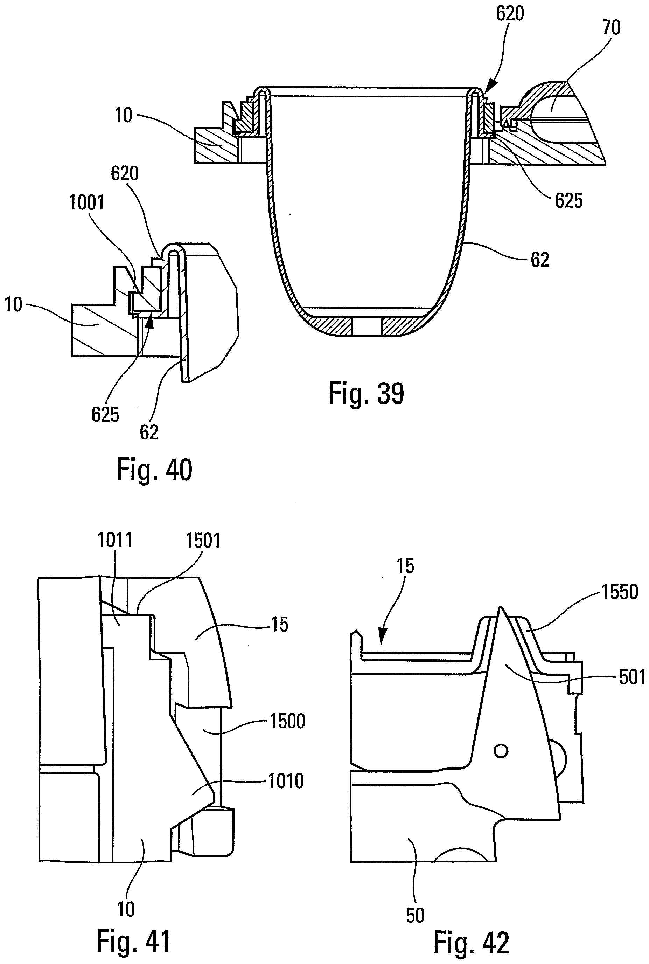

Les figures 33 et 34 montrent une autre variante de réalisation, dans laquelle une poche ou diaphragme 62 forme la chambre d'air 61. Cette poche 62 est reliée à l'embout buccal 15 via un canal 151 avantageusement disposé autour du canal d'expulsion 152 relié à la chambre de distribution 70. La poche 62 est fixée à une tige reliée aux moyens de blocage 100, l'inhalation provoquant la déformation de la poche 62 et donc de la tige pour déplacer lesdits moyens de blocage 100. Les figures 39 et 40 montrent une variante de réalisation de la poche 62. Celle-ci, avantageusement réalisée en silicone, peut comporter un ourlet 620 adapté à former une étanchéité avec le corps 10, par exemple entre le corps 10 et l'embout buccal 15. Pour ce faire, l'ourlet 620 peut se prolonger par une bride 625, également en silicone, qui va être comprimée par un partie d'encliquetage 1001 du corps 10 pour réaliser l'étanchéité, et notamment éviter toute perte de charge dans l'écoulement d'inhalation.

La figure 41 montre un détail de la fixation de la partie formant l'embout buccal 15 sur le corps 10, selon un mode de réalisation particulier. L'embout buccal 15 peut comporter une fenêtre 1500 coopérant avec une projection inclinée 1010 du corps 10. Comme par ailleurs une partie supérieure 1011 du corps 10 se trouve coincée par un épaulement 1501 de l'embout buccal 15, la projection inclinée 1010 assure l'étanchéité de l'encliquetage.

La figure 42 montre une variante de réalisation dans laquelle la tige pivotante 50 qui supporte la roue de guidage 30 comporte une extension 501 qui vient sensiblement boucher un trou 1550 prévu dans l'embout buccal 15 en position d'inhalation. Ainsi l'écoulement d'inhalation, qui avant déplacement de la roue de guidage 30 et sa tige 50 passe en partie par le trou 1550, se trouve après déplacement de ces éléments et bouchage du trou 1550, et donc après ouverture du réservoir, principalement canalisé vers le vidage dudit réservoir couvert. Il s'en suit une meilleure efficacité lors de l'inhalation qui participe à assurer un vidage optimal du réservoir.

Selon un autre aspect avantageux de l'inhalateur, les réservoirs individuels ou blisters 21 sont formés sur une bande allongée 20, qui est stockée sous forme de bobine à l'intérieur du corps 10 du dispositif. Avantageusement, cette bande de blister enroulée 20 est maintenue par des parois internes dudit corps 10 sans que son extrémité « arrière » (dans le sens de déplacement de la bande de blisters 20) ne soit fixée par rapport audit corps 10, ce qui permet un assemblage plus aisé de cette bobine de bande de blister à l'intérieur du dispositif. La bande de blister 20 est déplacée par l'utilisateur avantageusement au moyen de la roue de guidage 30 qui présente avantageusement au moins un, de préférence plusieurs évidements 31 dont la forme correspond à celle des blisters. Ainsi, lorsque cette roue de guidage 30 tourne, elle entraîne la bande de blister 20. Aucun autre système d'entraînement n'est nécessaire pour déplacer les blisters 21 lors de chaque actionnement. Bien entendu en variante ou de manière additionnelle, on pourrait utiliser d'autres moyens d'avancée de bande de blisters, par exemple en prévoyant un profil sur les bords longitudinaux latéraux de la bande de blister ledit profil étant adapté à coopérer avec des moyens d'entraînement appropriés. De même, des trous ménagés le long des bords latéraux de la bande de blister pourraient également être utilisés pour faire avancer la bande de blister au moyen de roues dentées coopérant avec lesdits trous.

Les moyens de blocage 100 sont destinés à maintenir les moyens de support mobiles 50 en position initiale et empêcher un déplacement du réservoir 21 vers sa position d'ouverture jusqu'à ce que l'utilisateur inhale. Ces moyens de blocage 100 doivent pouvoir être libérés de manière sûre et fiable et aisée lors de l'inhalation de l'utilisateur, de sorte que le déplacement du réservoir vers les moyens d'ouverture est réalisé rapidement, de manière fiable et sans nécessiter un effort trop important. Selon les exemples de réalisation représentés sur les figures 9 à 12, ces moyens de blocage 100 peuvent comporter deux éléments 101, 102 interconnectés l'un dans l'autre. Une rotation selon la flèche B est appliquée au second élément 102 lorsqu'un seuil d'inhalation approprié est atteint, la rotation de ce second élément 102 provoquant alors la libération du premier élément 101, ce premier élément 101 étant par ailleurs soumis à la charge selon la flèche A des moyens de support mobiles 50, sous l'effet de l'élément élastique 51 qui avait été préchargé lors de l'ouverture du dispositif. Le premier élément 101 pourrait être monté pivotant autour d'un point de pivot 109 disposé entre ses deux extrémités, une première extrémité subissant la charge des moyens de support mobiles 50 selon la flèche A, et l'autre extrémité coopérant avec le second élément 102. Dans cet exemple, représenté sur les figures 9 et 10, lorsque le second élément 102 a tourné selon la flèche B suite à une inhalation, l'extrémité du premier élément 101 qui était bloquée par le second élément 102 se déplace donc dans une direction (flèche C) opposée à la charge (flèche A). En variante, représenté sur les figures 11 et 12, on pourrait également utiliser une tige ou barre rotative 102 coopérant avec une cheville 101 exerçant une force axiale (flèche A) sur ladite tige 102 lorsque le dispositif est préchargé. Cette barre rotative 102 présente avantageusement une forme appropriée pour permettre à ladite tige chargée 101 de se déplacer librement dans la direction A de la force exercée sur elle par les moyens de support mobiles 50 préchargés lorsque ladite barre rotative 102 a tourné d'un certain angle. Par exemple, comme représenté sur la figure 11, la barre rotative 102 comporte une partie inférieure 107 de diamètre supérieur à la partie supérieure 108, de sorte qu'après une rotation de 90°, l'épaulement 106 formé entre ces deux parties 107 et 108 pousse la tige axiale 101 de manière à la décentrer par rapport à l'axe central de la barre rotative 102, de sorte que la tige axiale 101 va glisser le long de cette barre rotative 102 et pouvoir se déplacer vers le bas sur les figures 11 et 12 dans le sens de la flèche A pour permettre le déplacement des moyens de support mobiles 50 vers la position d'ouverture du réservoir. D'autres variantes de réalisation sont évidemment envisageables pour réaliser le blocage et libérer ce blocage lors de l'inhalation de l'utilisateur.

Selon encore un autre aspect de l'inhalateur, des moyens de comptage ou d'indication de doses 120 sont également prévus. Ces moyens peuvent soit comporter des chiffres ou symboles 125 inscrits directement sur la bande de blister 20 et visibles à travers une fenêtre appropriée 19 dans le corps 10 du dispositif. En variante, on pourrait envisager d'utiliser un disque rotatif 121 comportant les chiffres ou symboles 125, par exemple inscrits en forme en spirale sur ce disque. Dans ce cas, un élément coulissant (non représenté) avec une fenêtre appropriée pourrait être en prise avec une piste en spirale prévue sur ce disque 121 afin d'afficher le chiffre ou symbole 125 pertinent pour la dose en cours. Enfin, des indicateurs comportant des roues rotatives, par exemple une roue des unités et une roue des dizaines pourraient également être envisagées. D'autres variantes sont aussi envisageables, telles que l'utilisation de deux disques rotatifs superposés, ou un seul disque avec les chiffres inscrits autour de sa périphérie.

Après ouverture d'un ou plusieurs blister(s), la partie de bande de blister 35 avec les réservoirs vidés doit pouvoir être stockée de manière aisée et non encombrante dans le dispositif. Avantageusement, cette bande de blister usée 35 s'enroule automatiquement sur elle-même pour à nouveau former une bobine. Avantageusement, l'extrémité de cette bande de blister usée 35 (l'extrémité « avant » de la bande de blisters 20) peut être fixée à un élément ou arbre rotatif 150 qui accompagne chaque déplacement de la bande de blister en tournant d'un angle correspondant. Ceci favorise l'enroulement de la bande de blister usée 35 sur elle-même. De manière avantageuse, cet arbre 150 ne provoque aucune traction ni aucune autre force d'entraînement sur la bande de blister 20 mais ne fait que guider la rotation de son extrémité pour réaliser l'enroulement de la partie de bande usée 35.

Selon encore un aspect avantageux de l'inhalateur, des moyens d'aplatissement 160 de blisters usés peuvent être utilisés pour aplatir les blisters 21 une fois que la dose qu'ils contenaient a été vidée. Ceci permet de diminuer le volume de stockage de la bande de blister usée 35. Ces moyens d'aplatissement 160 pourraient être constitués de deux cylindres entre lesquels passe la bande de blister usée 35. Ces cylindres 160 pourraient être lisses ou présenter un profil approprié (par exemple cannelé, comme visible sur la figure 25) sur leur surface périphérique pour fournir une efficacité optimale tout en nécessitant une force minimale pour réaliser cet aplatissement de blisters utilisés ou vides.

Selon encore un autre aspect de l'inhalateur, les moyens d'ouverture 40 des réservoirs 21 comportent des moyens de perçage et/ou coupage 41. Ces moyens de perçage et/ou coupage 41 ont de préférence une forme appropriée de telle sorte que les parties de parois découpées 24 du blister 21 se plient vers l'intérieur du blister sans recouvrir les ouvertures 25 formées par ces moyens de perçage et/ou de coupage 41. Les figures 13 à 16 montrent un cycle d'ouverture et d'expulsion de la dose d'un blister. Ces moyens de perçage et/ou de coupage 41 comportent au moins deux extrémités de perçage opposées 42, 43 qui sont séparées l'une de l'autre par un écart approprié 44. Ces moyens de perçage et/ou de coupage 41 créent un pliage central avec des parties de parois découpées 24 permettant aux ouvertures formées 25 de ne pas du tout être recouvertes par une quelconque partie de parois découpée 24. Avantageusement, comme représenté sur les figures 13 à 17, chaque extrémité de perçage 42, 43 est en forme de coupelle et est formée par une partie de cylindre creux partiellement découpé ayant des bords coupants. Avantageusement, l'air entrant (flèche E) pénètre dans le blister ouvert 21 à l'extérieur desdits moyens de perçage et/ou de coupage 41, comme visible sur la figure 5. Ceci peut par exemple être obtenu grâce au fait que les moyens de support mobiles 50 se déplacent vers la position d'ouverture du réservoir selon une ligne courbe. Ce déplacement selon une courbe provoque une ou des ouverture(s) 25 légèrement plus large(s) que les dimensions de l'élément de perçage et/ou de coupage 41. Ceci permet donc à de l'air d'inhalation de s'écouler à l'extérieur dudit élément de perçage et/ou de coupage 41 pour pénétrer à l'intérieur du blister 21. Si nécessaire, des moyens spécifiques pour réaliser une ouverture à l'extérieur dudit élément de perçage et/ou de coupage 41 pourraient également être prévus, tels que par exemple des nervures 410 ou tout autre profil externe approprié sur ledit élément de perçage et/ou de coupage 41 (voir figures 29 à 32). De préférence, l'écoulement d'air sortant chargé avec la poudre (flèche S) sort du réservoir ou blister 21 à travers l'intérieur creux desdits moyens de perçage et/ou de coupage 41, comme représenté sur la figure 16. Eventuellement, comme représenté sur la figure 18, à la fois l'écoulement d'air entrant (flèche E) et l'écoulement d'air sortant chargé avec la poudre (flèche S) pourraient passer à travers des canaux creux respectifs prévus à l'intérieur des moyens de perçage et/ou de coupage 41. La forme spécifique de l'élément de perçage et/ou de coupage 41 tel que représentée sur les figures fournit une découpe du type « Louvre » qui fournit tous les avantages susmentionnés, et en particulier qui évite que les trous 25 créés par le perçage ne soient obturés même partiellement par des parties de parois percées 24. Ceci permet d'assurer un vidage le plus complet possible du blister 21, et fournit donc une efficacité maximale pour le distributeur. De même, la reproductibilité du dosage est optimale, la même quantité de poudre étant à chaque fois expulsée grâce au dispositif de l'invention.

Les figures 29 et 30 montrent une variante de réalisation de l'élément de perçage et/ou coupage 41, dans lequel une pointe centrale 420 sépare l'extérieur de l'élément en plusieurs canaux, en l'occurrence quatre. Des nervures externes 410 sont prévues au niveau du bord externe pour créer des ouvertures latérales permettant l'entrée de l'écoulement d'inhalation dans le réservoir.

Les figures 31 et 32 montrent une autre variante de réalisation, assez proche de celle des figures 13 à 17, à savoir avec deux extrémités de perçage 42, 43 séparées par un écart 44 adapté à loger au moins partiellement la partie de paroi découpée du blister. Ici, les ouvertures sont disposées dos à dos, contrairement à la figure 17 où elles étaient face à face. Avantageusement, des nervures externes 410 sont également prévues au niveau du bord externe.

En référence à la figure 26, il est représenté un deuxième mode de réalisation de l'inhalateur. Ce second mode de réalisation diffère principalement du premier mode de réalisation des figures 1 à 8 par la forme extérieure du dispositif et par la forme différente des moyens élastiques 51 permettant de précharger le dispositif lors de son ouverture. Dans ce second mode de réalisation, le corps 10 ne comporte qu'un seul élément de capot 11 qui est destiné à s'ouvrir pour charger le dispositif. Lors de cette ouverture, une lame élastique 51 est déformée par ledit élément de capot 11 qui s'ouvre fournissant ainsi la charge qui était fournie pas le ressort 51 dans l'exemple précédent. A nouveau, cette lame élastique 51 sollicite les moyens de support mobiles 50 en direction de la position d'ouverture du réservoir dans laquelle le réservoir 21 est déplacé contre les moyens d'ouverture 40, mais des moyens de blocage appropriés 100, tels que ceux décrits précédemment ou autres, sont prévus pour empêcher ce déplacement jusqu'à ce que l'utilisateur inhale. L'inhalation de l'utilisateur débloque lesdits moyens de blocage 100 et permet donc l'ouverture du réservoir 21 et la distribution automatique de la dose qu'il contient grâce aux moyens d'ouverture 40, et son expulsion dans les poumons de l'utilisateur grâce à la chambre de distribution 70 prévue en amont de l'embout buccal 15. Les moyens de support mobiles 50 peuvent être réalisés sous la forme d'une pièce plastique déformable incorporant d'une part la lame élastique 51, un élément de sollicitation 50 coopérant avec la roue de guidage 30, et une ou deux extension(s) 52, 53, adaptés à coopérer avec le corps 10 pour permettre la charge de la lame élastique 51. Dans l'exemple représenté, une première extension 52 coopère avec une roue dentée 200 pouvant former cliquet anti-retour et système d'enroulement de bande de blisters usée 35. Une seconde extension 53 peut coopérer avec la chambre de distribution 70, ou être relié d'une manière quelconque aux moyens de déblocage 100.

La figure 27 montre encore un mode de réalisation, qui diffère principalement de celui de la figure 26 par une autre forme des moyens élastiques 51 pour précharger le dispositif lors de l'ouverture du dispositif. La forme du corps 10 de l'inhalateur est également légèrement modifiée même si la différence est moins importante que par rapport au premier mode de réalisation des figures 1 à 8. La forme différente du moyen élastique 51 permettant de précharger le dispositif lors de l'ouverture du capot 11 diffère légèrement de celle représentée sur le deuxième mode de réalisation mais sa fonction est strictement identique et ne sera donc pas plus amplement décrite ici. Dans cet exemple particulier, les moyens de support mobiles 50 sont solidaires d'une lame élastique 51 déformée lors de l'ouverture du dispositif, et une seconde pièce 54, élastique ou non, peut être prévue pour réaliser l'enroulement de la bande de blisters usée 35 et/ou le déblocage des moyens de blocage 100. Il est à noter que ces moyens de blocage 100 ne sont pas représentés sur les figures 26 et 27, et que leur actionnement peut être réalisé d'une quelconque manière appropriée.

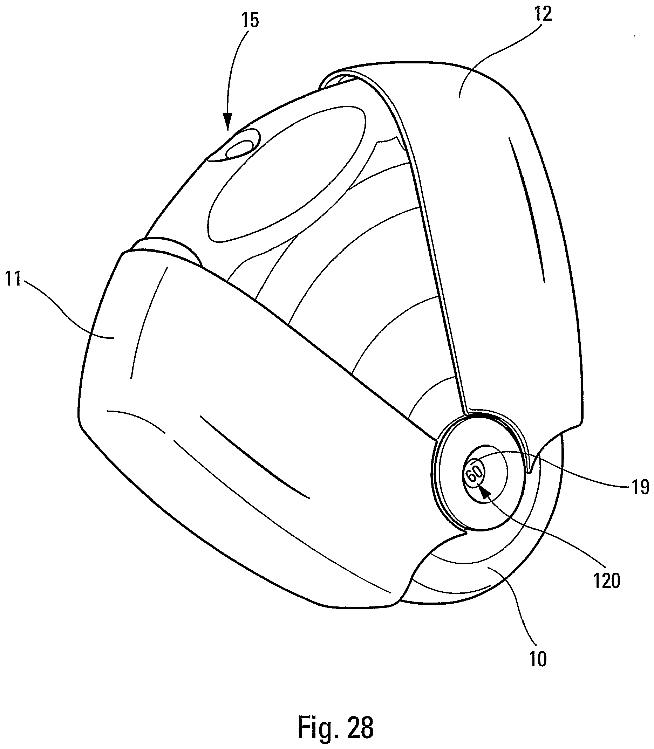

La figure 28 montre une vue externe d'un autre mode de réalisation avantageux de l'inhalateur, assez proche de ceux des figures 1 à 8, et qui en diffère notamment par une forme différente de l'embout buccal 15.

Dans tous les modes de réalisation décrits ci-dessus, la bande de blister est formée par une bande présentant deux extrémités. En variante, on pourrait utiliser une bande continue. D'autres modifications sont également possibles sans sortir du cadre de la présente invention.

La présente invention permet donc de fournir un inhalateur de poudre sèche qui procure notamment les fonctions suivantes :
▪ une pluralité de doses individuelles de poudre stockées dans des réservoirs individuels étanches, par exemple 60 doses stockées sur une bande enroulée en bobine ;
▪ la poudre libérée au moyen d'un perçage actionné par l'inhalation de l'utilisateur, ce perçage du blister étant réalisé au moyen d'un système de détection d'inhalation couplé à un système de libération préchargé ;
▪ des moyens d'entraînement de forme appropriée en prises avec les blisters pour réaliser le déplacement de la bande de blister à chaque actionnement, et amener un nouveau réservoir dans une position dans laquelle il est destiné à être ouvert par les moyens d'ouverture appropriés ;
▪ un indicateur de doses relié mécaniquement au mouvement de la bande de blister.

D'autres fonctions sont également fournies par le dispositif de l'invention tel que cela a été décrit précédemment. Il est à noter que les différentes fonctions, même si elles ont été représentées comme prévues simultanément sur les différents modes de réalisation de l'inhalateur, pourraient être mises en oeuvre séparément les unes des autres. En particulier, le mécanisme de déclenchement par inhalation pourrait être utilisé indépendamment du type de moyens d'ouverture de réservoir, indépendamment de l'utilisation d'un indicateur de doses, indépendamment de la manière dont les réservoirs individuels sont arrangés les uns par rapport aux autres, etc. Les moyens d'armement et le système de déclenchement par l'inhalation pourraient être réalisés différemment. Il en est de même des autres parties constitutives du dispositif.

L'inhalateur de l'invention, incorporant toutes ou parties des fonctions décrites, s'avère fournir des performances supérieures à celles des dispositifs existants. En particulier, l'inhalateur de l'invention fournit un taux de vidage des réservoirs d'au moins 90% à chaque actionnement. Avantageusement, ce taux de vidage, correspondant au pourcentage du produit fluide expulsé hors d'un réservoir ouvert lors d'un actionnement du dispositif, est supérieur à 95%, de préférence même supérieur à 97%. Ce taux de vidage élevé est notamment même supérieur aux performances obtenues avec des inhalateurs actifs qui sont généralement plus efficaces que les inhalateurs passifs, et dans lesquels ce n'est pas l'écoulement d'inhalation qui vide le blister et expulse la dose mais un écoulement d'air comprimé libéré au moment de l'inhalation. Il garantit une efficacité maximale du dispositif de l'invention. Couplé à l'ouverture déclenchée par l'inhalation, ce taux de vidage élevé garantit une distribution optimale du produit fluide, en l'occurrence la poudre, dans les poumons de l'utilisateur. Le tableau ci-dessous illustre des mesures réalisées avec un mélange Budésonide / lactose à 1.17% en poids, avec divers débits d'écoulement correspondant à des débits typiques d'écoulements d'inhalation. Ainsi, trois mesures ont été réalisées avec des débits respectifs d'environ 7.5 l/min, 10 l/min et 15 l/min. Les mesures ont consisté à mesurer la quantité de poudre restant dans le blister après vidage par l'écoulement d'air, et donc, en comparant avec la quantité de poudre introduite dans le blister, le taux de vidage de celui-ci. Ces mesures démontrent l'efficacité très élevée du dispositif de l'invention, le taux de vidage dans cet exemple étant systématiquement au moins égal à 97%.

| **Débit (l/min)** | **Poudre introduite dans blister (mg)** | **Poudre restant dans blister (mg)** | **Quantité de poudre émise du blister (mg)** | **% de dose émise** | |
|---|---|---|---|---|---|
| 7,5 | 10,63 | 0,00 | 10,63 | 1,00 | 0,98 |
| 7,5 | 10,18 | 0,13 | 10,05 | 0,99 | |
| 7,4 | 9,78 | 0,32 | 9,46 | 0,97 | |
| 10,0 | 9,86 | 0,31 | 9,55 | 0,97 | 0,99 |
| 10,0 | 10,38 | 0,00 | 10,38 | 1,00 | |
| 9,9 | 9,77 | 0,00 | 9,77 | 1,00 | |
| 15,0 | 10,01 | 0,00 | 10,01 | 1,00 | 1,00 |
| 14,5 | 9,76 | 0,04 | 9,72 | 1,00 | |
| 14,5 | 10,04 | 0,02 | 10,02 | 1,00 | |

Par ailleurs, l'invention fournit également une meilleure régularité de vidage des réservoirs lors d'actionnements successifs. Ainsi, en se référant par exemple à 10 réservoirs d'une bande de blisters, il s'avère que le taux de vidage varie de moins de 15%, avantageusement de moins de 10%, de préférence de moins de 5% d'un réservoir à l'autre. Cette meilleure régularité garantit une meilleure reproductibilité de la dose et donc aussi une meilleure efficacité du dispositif de l'invention.

Diverses modifications sont également possibles pour un homme du métier sans sortir du cadre de la présente invention tel que défini par les revendications annexées.

## Revendications

1. Dispositif de distribution de produit fluide, comportant au moins un réservoir individuel (21) contenant une dose unique de poudre, des moyens d'ouverture (40) étant prévus pour ouvrir un réservoir individuel (21) à chaque actionnement du dispositif, permettant à l'utilisateur d'inhaler la dose contenue dans ledit réservoir ouvert, la poudre contenue dans un réservoir ouvert étant expulsée par un écoulement d'air créé par l'inhalation de l'utilisateur, lesdits moyens d'ouverture (40) comportant des moyens de perçage et/ou de coupage (41) adaptés à découper une paroi de fermeture (23) du réservoir (21) de telle sorte que la ou les partie(s) découpée(s) (24) n'obstrue(nt) pas la ou les ouverture(s) formée(s) (25), lesdits moyens de perçage et/ou de coupage (41) comportent deux extrémités de perçage opposés (42, 43) séparées l'une de l'autre par un écart (44) pour former la ou les partie(s) découpée(s) (24) en un pliage central, lesdits moyens d'ouverture (40) étant déclenchés par l'inhalation de l'utilisateur, de sorte qu'au moins 90%, avantageusement au moins 95%, de préférence au moins 97%, de la poudre contenue dans ledit réservoir ouvert est expulsée hors dudit réservoir lors de l'inhalation.

2. Dispositif selon la revendication 1, dans lequel une chambre de distribution (70) est prévue pour recevoir la dose de produit contenue dans un réservoir (21) après son ouverture, ladite chambre de distribution (70) étant reliée d'une partie auxdits moyens d'ouverture (40) et d'autre part à un orifice de distribution (79) relié à un embout d'inhalation (15).

3. Dispositif selon la revendication 2, dans lequel ladite chambre de distribution (70) contient au moins une bille mobile (75).

4. Dispositif selon l'une quelconque des revendications précédentes, dans lequel les réservoirs (21) sont réalisés sous la forme d'une bande allongée (20) comportant plusieurs réservoirs individuels (21) disposés les uns à la suite des autres.

5. Dispositif selon l'une quelconque des revendications précédentes, comprenant des moyens de support mobiles (50) adaptés à supporter et déplacer un réservoir individuel (21) contre des moyens d'ouverture (40) à chaque actionnement.

6. Dispositif selon la revendication 5, dans lequel lesdits moyens de support mobiles (50) comportent une roue de guidage qui coopère avec des moyens de butée (350) adaptés à déterminer la position de distribution desdits moyens de support mobiles (50) après inhalation.

## Patentansprüche

1. Ausgabevorrichtung für ein fluides Produkt, aufweisend mindestens einen einzelnen Behälter (21), der eine einzige Dosis Pulver enthält, wobei Öffnungsmittel (40) vorgesehen sind, um bei jeder Betätigung der Vorrichtung einen einzelnen Behälter (21) zu öffnen, was es dem Benutzer ermöglicht, die in dem geöffneten Behälter enthaltene Dosis zu inhalieren, wobei das in einem Behälter enthaltene Pulver durch einen Luftstrom ausgestoßen wird, der durch die Inhalation des Benutzers erzeugt wird, wobei die Öffnungsmittel (40) Durchstech- und/oder Schneidemittel (41) aufweisen, die dazu geeignet sind, eine Verschlusswand (23) des Behälters (21) derart abzuschneiden, dass der abgeschnittene Teil oder die abgeschnittenen Teile (24) die gebildete(n) Öffnung(en) (25) nicht versperrt (versperren), wobei die Durchstech- und/oder Schneidemittel (41) zwei gegenüberliegende Durchstechenden (42, 43) aufweisen, die voneinander um einen Abstand (44) beabstandet sind, um den oder die abgeschnittenen Teil(e) mittig zu falten, wobei die Öffnungsmittel (40) durch die Inhalation des Benutzers derart ausgelöst werden, dass mindestens 90 %, vorteilhafterweise mindestens 95 %, vorzugsweise mindestens 97 % des in dem offenen Behälter enthaltenen Pulvers bei der Inhalation aus dem Behälter ausgestoßen werden.

2. Vorrichtung nach Anspruch 1, wobei eine Ausgabekammer (70) vorgesehen ist, um die in dem Behälter (21) enthaltene Produktdosis nach dessen Öffnung aufzunehmen, wobei die Ausgabekammer (70) einerseits mit den Öffnungsmitteln (40) und andererseits mit einer Ausgabeöffnung (79) verbunden ist, die mit einem Inhalationsansatz (15) verbunden ist.

3. Vorrichtung nach Anspruch 2, wobei die Ausgabekammer (70) mindestens eine bewegliche Kugel (75) enthält.

4. Vorrichtung nach einem der vorhergehenden Ansprüche, wobei die Behälter (21) in Form eines langgestreckten Streifens (20) ausgebildet sind, der mehrere einzelne Behälter (21) enthält, die hintereinander angeordnet sind.

5. Vorrichtung nach einem der vorhergehenden Ansprüche, aufweisend mobile Trägermittel (50), die dazu geeignet sind, bei jeder Betätigung einen einzelnen Behälter (21) zu tragen und gegen die Öffnungsmittel (40) zu verschieben.

6. Vorrichtung nach Anspruch 5, wobei die mobilen Trägermittel (50) ein Führungsrad aufweisen, welches mit Anschlagmitteln (350) zusammenwirkt, die dazu geeignet sind, die Ausgabeposition der mobilen Trägermittel (50) nach der Inhalation zu bestimmen.

## Claims

1. A fluid dispenser device including at least one individual reservoir (21) containing a single dose of powder, opening means (40) being provided for opening an individual reservoir (21) each time the device is actuated, thereby enabling the user to inhale the dose contained in said open reservoir, the powder contained in an open reservoir being expelled by a flow of air created by the user inhaling, said opening means (40) comprising perforator and/or cutter means (41) that are adapted to cut a closure wall (23) of the reservoir (21) in such a manner that the cut portion(s) (24) does/do not obstruct the opening(s) (25) that is/are formed, said perforator and/or cutter means (41) comprising opposite perforator ends (42, 43) separated from each other by a gap (44) to form the cut portion(s) (24) into a central fold, said opening means (40) being triggered by the user inhaling, in a manner that at least 90%, advantageously at least 95%, preferably at least 97% of the powder contained in said open reservoir is expelled from said reservoir during inhalation.

2. A device according to claim 1 in which a dispenser chamber (70) is provided for receiving the dose of fluid contained in a reservoir (21) after said reservoir has been opened, said dispenser chamber (70) being connected firstly to said opening means (40), and secondly to a dispenser orifice (79) that is connected to an inhaler endpiece (15).

3. A device according to claim 2, in which said dispenser chamber (70) contains at least one movable bead (75).

4. A device according to any preceding claim, in which the reservoirs (21) are made in the form of an elongate strip (20) comprising a plurality of individual reservoirs (21) disposed one behind the other.

5. A device according to any preceding claim, including movable support means (50) that are adapted to support and to displace an individual reservoir (21) against opening means (40) on each actuation.

6. A device according to claim 5, in which said movable support means (50) comprise a guide wheel which cooperates with abutment means (350) that are adapted to determine the dispensing position of said movable support means (50) after inhalation.
